# EUROPÄISCHE PATENTSCHRIFT

(11) **EP 3 209 229 B1**
(45) Veröffentlichungstag und Bekanntmachung des Hinweises auf die Patenterteilung: **28.07.2021**
(21) Anmeldenummer: 16770206.7
(22) Anmeldetag: 01.09.2016
(51) Int. Cl.: A61B 17/70, A61B 17/86, A61B 90/00

(54) **VORRICHTUNG ZUM BEFESTIGEN EINES STABS AN EINEM KNOCHEN**
DEVICE FOR SECURING A ROD TO A BONE
DISPOSITIF DE FIXATION D'UNE TIGE SUR UN OS

(30) Priorität: 06.10.2015 DE 102015012909; 30.06.2016 DE 202016004114 U
(43) Veröffentlichungstag der Anmeldung: 30.08.2017
(73) Patentinhaber: Joimax GmbH, 76227 Karlsruhe (DE)
(72) Erfinder: RIES, Wolfgang, 76351 Linkenheim-Hochstetten (DE); SCHENDZIELORZ, Lars, 76351 Linkenheim-Hochstetten (DE); DÜRR, Alexander, 71732 Tamm (DE)
(74) Vertreter: Lichti - Patentanwälte Partnerschaft mbB
(86) Internationale Anmeldenummer: PCT/EP2016/001479
(87) Internationale Veröffentlichungsnummer: WO 2017/059941

(56) Entgegenhaltungen:
- EP-A1- 2 363 086
- WO-A2-2009/106733
- US-A1- 2005 240 180
- US-A1- 2012 083 850
- US-A1- 2014 236 235

## Beschreibung

Die Erfindung betrifft eine Vorrichtung zum Befestigen eines Stabs an einem Knochen nach dem Oberbegriff des Anspruchs 1.

Insbesondere bei beschädigten Bandscheiben aber auch sonstigen Schädigungen der Wirbelsäule ist es oft notwendig, zwei oder mehr benachbarte Wirbelkörper durch Spondylodese (Wirbelkörperverblockung) zu versteifen. Hierzu werden in die Wirbelkörper Pedikelschrauben eingeschraubt, die rückwärtig proximal eine Tulpe oder Stablagerung tragen. Ein jeweils eine Pedikelschraube bzw. Tulpen in mehreren Wirbeln verbindenden Quer-Stab ist in den Tulpen mittels Spannschrauben verspannt.

Dabei ist es zwar bekannt die Pedikelschrauben mit einem Kugelkopf oder einem Teilkugelkopf auszubilden und zwischen Stab und Pedikelschraubenkopf ein Andruckelement vorzusehen, womit Tulpenkopf und Pedikelschraube mono- oder polyaxial relativ zueinander unter einem Winkel ausgerichtet werden können. Andruckschrauben wirken aber immer unmittelbar auf dem (Quer-)Stab. Krümmungen der Wirbelsäule (Kyphose - Brust, Lordose - Lende) können zwar durch entsprechend gebogene Verbindungsstäbe berücksichtigt bzw. angepasst werden. Dennoch können bei den bekannten Vorrichtungen unerwünschte Spannungen auftreten, da die den Stab im Tulpenkopf festspannende Spannschraube immer senkrecht, d.h. flächig in Richtung ihrer eigenen Symmetrieachse gegen den Stab einwirkt und diesen derart senkrecht auch im Tulpenkopf verspannt.

Die EP 2 363 086 A1 zeigt eine transpedikuläre Schraube mit einem Kopf 1 und einem mit einem Schraubgewinde versehenen Schaft, wobei der Kopf 1 mit dem Schaft der Pedikelschraube einstückig und damit starr verbunden ist und im Kopf 1 die Wandung durch eine diametrale Schlitzung unterbrochen ist, durch die sich der Befestigungs- oder Verbindungsstab 7 erstrecken kann. Distal zu dieser ist ein "unteres Teil" (lower part 3) vorgesehen, das zwar relativ zur Hauptachse - der Pedikelschraube - eine von einem rechten Winkel abweichende Ausrichtung ermöglicht und zwar aufgrund einer teilzylindrischen dem Verbindungsstab abgewandten Unterseite des Elements 3. Eine Schwenkbarkeit im dritten (rotatorischen) Freiheitsgrad ist aber offensichtlich nicht gegeben, da die Seitenwandungen des Teils 3 ebenso wie die des Teils 4 offensichtlich Plan sind; hierzu ist auch nichts gesagt. Insbesondere ist nicht gesagt, dass der Durchmesser des Andruckelements an mindestens einer seiner Stirnseiten gegenüber dem Innendurchmesser der Tulpe reduziert ist. Insbesondere weist das untere Teil 3 der D1 an seiner proximalen Stirnseite seine jeweils weiteste Erstreckung in radialer Richtung auf.

Der Erfindung liegt die Aufgabe zugrunde, eine gattungsgemäße Vorrichtung dahingehend weiterzuentwickeln, dass sie weitergehende Freiheitsrade und insbesondere einen für einen Patienten weniger belastenden und weniger gefährlichen Einsatz ermöglicht.

Erfindungsgemäß wird die oben genannte Aufgabe mit einer gattungsgemäßen Vorrichtung gelöst, die die kennzeichnenden Merkmale des Anspruchs 1 aufweist.

Erfindungsgemäß ist also zusätzlich zur Spannschraube eine zwischen dieser und den in dem Tulpenkopf zu verspannenden (Quer-)Stab angeordnete Stabklemme vorgesehen, die darüber hinaus relativ zur Spannschraube schwenkbar ist. Hierdurch können bei der Spondylodese mittels eines solchen Verbindungsstabes insbesondere über mehrere zu versteifende Wirbelkörper auftretende Unregelmäßigkeiten ausgeglichen werden.

Es ergibt sich so nach Festspannen immer unabhängig vom Durchmesser des Stabes (im vorgegebenen Bereich von insbesondere 5 mm bis 6 mm) zumindest ein linienförmiger Kontakt, eine Kontaktlinie, und damit eine zuverlässige Verbindung und gleichmäßige Kraftübertragung. Ein lediglich punktförmiger Kontakt ist ausgeschlossen.

Insbesondere beinhaltet die Erfindung damit auch ein System aus mindestens einer erfindungsgemäßen Vorrichtung und einem (Verbindungs)-Stab, bei dem der eingespannte Stab zwischen Spannschraube und Stabklemme zu jeder der beiden zumindest über eine Kontaktlinie in Verbindung steht, gegebenenfalls auch flächig, (aber eben nicht lediglich punktförmig). Dies gilt für Verbindungsstäbe mit unterschiedlichen Durchmessern (in einem beschränkten Bereich), vorzugsweise zwischen 5 mm und 6 mm.

Eine bevorzugte Ausgestaltung der erfindungsgemäßen Vorrichtung zeichnet sich dadurch aus, dass die Stabklemme und Spannschraube einander zugewandte schräg zu einer Hauptachse (A) ausgerichtete beidseitige Kugelzonen aufweisen, über die sie relativ verschwenkbar sind. Die Kugelzonen sind bevorzugt in dem Mantelbereich von Spannschraube und Stabklemme ausgebildet. Vorzugsweise weist die Spannschraube an ihrem distalen Ende der Stirnseite eine von außen distal nach innen proximal schräg verlaufende ringförmige Stirnseite in Form einer Kugelzone auf (als Mantel einer Kugelschicht) und die Stabklemme eine korrespondierende proximale Stirnseite in Form einer Kugelzone auf.

"Proximal" bezeichnet einen einem Operateur oder Benutzer - axial - zugewandten, distal einen diesem abgewandten und damit einem Patienten zugewandten bzw. in diesem liegenden Bereich der Vorrichtung bei ihrem Einsatz.

In bevorzugter Weiterbildung ist dabei ein Spannschraube und Stabklemme verbindender Stabklemmenadapter vorgesehen, wobei der Stabklemmenadapter ein Ringteil mit proximalen nach außen radial gerichtete Vorsprüngen ist, die in radiale Einschnitte in der Spannschraube unter Spiel eingreifen, wobei insbesondere distale, ebenfalls radial nach außen gerichtete Vorsprünge vorgegeben sind, die in radiale Einschnitte der Stabklemme eingreifen.

Proximal hinter der Stirnseite der Spannschraube ist eine radiale Ringnut als Hinterschnitt ausgebildet. Eine Stabklemme für den einzuspannenden Stab weist an ihrem zur Spannschraube hin gewandten proximalen Ende eine zu einer Hauptlängsachse ebenfalls schräg verlaufende, der Stirnseite entsprechende Stirnseite auf. Auch die Stabklemme ist an der Innenseite ihres Mantels mit einer radialen Ringnut versehen. In die Ringnut greifen radial nach außen gebildete Vorsprünge des ringförmigen Stabklemmenadapters ein, um so die Stabklemme an der Spannschraube gegen Herausfallen zu sichern.

In einer bevorzugten Ausgestaltung der erfindungsgemäßen Vorrichtung ist vorgesehen, dass Spannschraube und Stabklemme einander hintergreifende Hinterschneidungen aufweisen, wodurch eine schwenkbare Verbindung zwischen Spannschraube und Stabklemme gegeben ist.

Hierdurch wird erreicht, dass Spannschraube und Stabklemme trotz ihrer relativen Schwenkbeweglichkeit nicht verlierbar miteinander verbunden sind. Zum Schaffen der Hinterschneidung ist in erfindungsgemäßer Ausgestaltung vorgesehen, dass die Hinterschneidung der Spannschraube durch einen Ringvorsprung gebildet ist und/oder dass die Hinterschneidung der Stabklemme an mehr als zwei über den Umfang angeordneten elastischen Fingern ausgebildet sind. Die Zahl der Ringe und Hinterschneidungen an diesen beträgt vorzugsweise vier bis acht. Durch letztere Ausgestaltung können die zunächst separat hergestellten Spannschraube und Stabklemme miteinander durch axiales Gegeneinanderdrücken verbunden werden, wobei die Finger der Spannklemme hinter den Ringvorsprung der Spannschraube zurückweichen und ihre Vorsprünge anschließend den Ringvorsprung der Spannschraube bzw. deren Hinterschneidung hintergreifen. Trotz der relativ zueinander beweglichen und insbesondere schwenkbaren Verbindung ist damit eine Verliersicherheit beider Teile gegeben.

In weiterer bevorzugter Ausgestaltung der Erfindung ist vorgesehen, dass die Spannschraube einen von der Hinterschneidung fort sich verjüngenden Innenkonus aufweist. Damit wird erreicht, dass auch bei einer nicht axialen Ausrichtung, d.h. einem Verschwenken oder Verkippen zwischen Spannschraube und Stabklemme immer eine feste Radialposition zwischen beiden Teilen gegeben ist, da der zum proximalen oder verjüngten Bereich des Innenkonus der Spannschraube sich bewegende Bereich der Stabklemme eben durch die verjüngte konische Ausführung zur Seite gedrückt wird und der gegenüberliegende im erweiterten oder distalen Bereich der Ausnehmung der Spannschraube entsprechend zur Seite in diesem Bereich ebenfalls an der Umfangswandung der Spannschraube anliegt.

Gemäß einer weiteren bevorzugten Ausgestaltung der erfindungsgemäßen Vorrichtung ist vorgesehen, dass die Stabklemme auf ihrer der Spannschraube abgewandten Seite eine teilkreisförmige Ausnehmung aufweist. Eine solche teilkreisförmige Ausnehmung weist eine Symmetrieachse senkrecht zur Hauptsymmetrieachse von Spannklemme und bei axial gestreckter Anordnung derselben mit der Symmetrieachse der Spannschraube auf. Die teilkreisförmige Ausnehmung der Stabklemme kann daher den Stab über einen Teil seines Umfanges umfassen.

In weiterer bevorzugter Ausgestaltung ist vorgesehen, dass die Spannschraube ein asymmetrisches Gewinde aufweist, wobei insbesondere eine zur Festschraubrichtung proximal gerichtete Flanke des Gewindes der Spannschraube sich unter einem Winkel von kleiner gleich 5°, insbesondere kleiner gleich 3°oder ungleich 0°, zu einer Radialebene zur Symmetrieachse der Spannschraube erstreckt und/oder eine in Festschraubrichtung (distal) gerichtete Flanke des Gewindes der Spannschraube sich unter einem von 90° abweichenden Winkel zur Symmetrieachse der Spannschraube, vorzugsweise um 30°, zu einer Radialebene erstreckt. Die Flanken sind dabei proximal zur senkrechten Längsachse der Schraube gerichtet. Gemäß einer weiteren bevorzugten Ausgestaltung ist vorgesehen, dass die Spannschraube auf ihrer der Stabklemme abgewandten Seite eine mehrzählig - rotationssymmetrische (nicht zylindrische) Vertiefung aufweist. Zu dieser proximalen nicht-zylindrischen Ausnehmung der Spannschraube kann so ein komplementär ausgebildetes Schraubenwerkzeug zum Einschrauben der Schraube in die Tulpe der Vorrichtung eingreifen.

Eine weitere Ausgestaltung der Erfindung ist dadurch gekennzeichnet, dass das Andruckelement kippbar in der Tulpe gelagert ist. Hierzu ist weiterhin vorgesehen, dass der Durchmesser des Andruckelements an mindestens einer seiner Stirnseiten gegenüber dem Innendurchmesser der Tulpe reduziert ist, so dass das Andruckelement innerhalb der Tulpe kippbar ist. Das Andruckelement der Tulpe verjüngt sich von seiner distalen zu seiner proximalen Stirnseite, so dass proximal ein radiales Spiel zwischen Innenwand der Tulpe und der Außenwand des Andruckelements gegeben ist oder dass die Innenwandung der Tulpe sich von der Höhe der proximalen zur Höhe der distalen Stirnseite des Andruckelements unter Gewährleistung eines radialen Spiels zwischen Andruckelement und Tulpe auf Höhe der distalen Stirnseite des Andruckelements erweitert.

Damit ist das der Stabklemme distal gegenüberliegende, den Stab zwischen dieser und sich einspannende Andruckelement in Verbindung bzw. Beziehung zum Innenraum der umgebenden Tulpe so ausgestaltet und/oder gelagert, dass das Andruckelement die durch die Spannvorrichtung ermöglichte Ausrichtung des eingespannten Stabs aufnimmt, ebenfalls ermöglicht und nicht behindert.

Eine erfindungsgemäße Befestigungsvorrichtung weist eine Pedikelschraube, ein Andruckelement und eine Tulpe auf. Sie hat weiter eine Spannvorrichtung mit einer Spannschraube und einer Stabklemme. Alle diese Teile sind kanuliert, d.h. sie weisen einen sich axial erstreckenden zentralen Hohlraum auf.

Die Tulpe hält die Pedikelschraube und dient nach Einschrauben letzterer in einem Knochen zur Lagerung eines Stabs. Die Tulpe ist also eine Stablagerung und wird auch als solche bezeichnet.

Die Tulpe ist proximal über eine Sollbruchstelle mit einer Tulpenverlängerung verbunden. Tulpe und Tulpenverlängerung weisen ein sich über beide erstreckendes Innengewinde und in der Wandung beider einander diametral gegenüberliegende Langlöcher auf.

Die Pedikelschraube hat einen Pedikelschraubenschaft mit einem selbstschneidenden Pedikelgewinde und am proximalen Ende einen Pedikelschraubenkopf, der von der Tulpe umfasst ist. Im distalen Bereich der Tulpe ist ein Andruckelement für den Stab als Widerlager angeordnet.

In bevorzugter Ausgestaltung ist vorgesehen, dass der Schraubenschaft der Pedikelschraube ein Zweifachgewinde aufweist, wobei insbesondere ein proximaler Bereich des Schraubenschaftes als Vierfachgewinde ausgebildet ist, vorzugsweise über eine Länge von ein Viertel des Pedikelschraubenschaftes. Hierdurch wird ein besserer Halt der Schraube im Knochen erreicht. Ein Zweifachgewinde weist zwei Schraubengänge auf. Diese sind ineinander gewunden.

Der Übergang von Schraubengewindevorsprung bzw. -zähnung zum Schraubenschaft ist abgerundet mit einem endlichen Krümmungsradius des Übergangs. Hierdurch wird die Stabilität der Schraube erhöht und ein Abbrechen der Schraubflanken verhindert.

Der Pedikelschraubenschaft ist mit Durchbrüchen um einen Mantel zu seinem inneren Lumen versehen. Durch ein Lumen der Schraube und Durchbrüche können insbesondere pastöse Massen, wie Knochenzement eingebracht werden. Wesentlich ist dabei, dass die Durchbrüche im distalen Bereich der Schraube vorhanden sind, damit sie nach Einbringen der Schraube innerhalb des Wirbelkörpers (und nicht im Knochenbereich) liegen, so dass Zement durch diese dann in das Innere des Wirbelkörpers zur Fixierung eingebracht werden kann.

Ein Außengewinde der Spannschraube ist auf ein Innengewinde der Tulpe abgestimmt. Ein in der Tulpe eingebrachter Stab ist zwischen der ihn von der proximalen Seite umgreifenden Stabklemme, auf die die Spannschraube wirkt, und dem distal angreifenden Andruckelement eingespannt, durch die der Stab mit Winkeln gleich oder auch ungleich 90°, insbesondere zur Vorrichtungsachse, eingespannt werden kann.

Bevorzugte konkrete Ausbildungen einzelner Elemente sind im Folgenden genannt. Am proximalen Ende der Spannschraube ist eine mehrzählige nicht-zylindrische Vertiefung zum formschlüssigen Eingriff mit einem Schraubwerkzeug zur Übertragung eines Drehmoments auf die Spannschraube vorgesehen. Die Außenseite der Spannschraube ist mit einem asymmetrischen Spannschraubengewinde, insbesondere in Form eines speziellen Sägengewindes, versehen.

Die Spannschraube weist proximal hinter ihrer distalen Stirnseite einen Ringvorsprung in Form eines sich von der distalen Stirnseite proximal erstreckenden Innenkonus auf, dem sich über eine Hinterschneidung proximal ein das Lumen der Spannschraube in proximaler Richtung verjüngender Innenkonus anschließt, der am distalen Endbereich der Vertiefung der Spannschraube radial einen größeren Durchmesser als die Vertiefung hat.

Die Stabklemme weist eine Reihe von in Umfangsrichtung nebeneinander angeordneten und in proximaler Richtung und radial nach innen gerichteten elastischen Fingern mit radial nach außen weisenden Nasen auf, die mit den Hinterschneidungen der Spannschraube zusammenwirken, indem die Nasen mit ihren Hinterschneidungen in der durch den Ringvorsprung gebildeten Hinterschneidung der Spannschraube einrasten. Die distale Stirnseite der Stabklemme hat eine teilzylindrische Klemmausnehmung zur Aufnahme des Querstabs.

Aufgrund der Dimensionierung des Innenkonus der Spannschraube ist eine Kippbewegung der Stabklemme in der Spannschraube möglich. Durch die Ausgestaltung des sich proximal verjüngenden Innenkonus befinden sich auch in gekippter Stellung der Stabklemme deren Nasen in Kontakt mit der Innenwand des Innenkonus. Auf diese Weise ist die radiale Festlegung der Stabklemme 6 in axial ausgerichteter und in gekippter Stellung gewährleistet.

Ein polyaxiales Andruckelement weist auf seiner proximalen Seite eine teilzylindrische Ausnehmung zum teilweisen Umfassen bzw. Aufnehmen des Stabs auf, während eine weitere halbkugel- oder kalottenförmige Ausnehmung an der distalen Stirnseite des Andruckelements vorgesehen ist, um einen kugelförmigen Schraubenkopf der Pedikelschraube zu erfassen.

Das - gegenüber dem Stab - distale - Andruckelement hat einen sich von seinem distalen Ende zum proximalen Ende hin verjüngenden Mantel, so dass zwischen diesem und der Innenwandung der Tulpe ein Spalt gebildet ist. Hierdurch ist das Verkippen des Andruckelements innerhalb der Tulpe gegenüber einer axial ausgebildeten zentrischen Stellung möglich.

Die teilzylindrische Ausnehmung weist einen Krümmungsradius derart auf, dass Stäbe mit unterschiedlichem Durchmesser, wie zwischen 5 mm und 6 mm, eingesetzt werden können.

Das Andruckelement hat an seiner Außenseite Andruckflächen für radial von außen durch die Tulpe einschraubbare Querschrauben zum Fixieren des Andruckelements innerhalb der Tulpe.

Die polyaxiale Pedikelschraube weist einen kugelförmigen Pedikelschraubenkopf auf, der in der kugelförmigen Ausnehmung am distalen Bereich des polyaxialen Andruckelements in allen Richtungen uneingeschränkt schwenkbar ist. Der Schraubenkopf und damit die Pedikelschraube 2 wird mittels des Andruckelements - unverlierbar - in der Tulpe gehalten, da diese an ihrem distalen Ende eine gegenüber dem Durchmesser des Schraubenkopfes verengte Öffnung aufweist.

Ein monoaxiales Andruckelement weist eine den Schraubenkopf der Pedikelschraube zugewandte flache kegelstumpfförmige distale Stirnseite mit einem kegelringförmigen Umfangsrand auf, der einen zentralen Durchbruch umgibt.

Bei der monoaxialen Ausrichtung sind Andruckelement und Pedikelschraube relativ zueinander verdrehbar, bleiben aber immer entlang einer Achse, die mit der Achse der Tulpe und der in dieser eingeschraubten Spannschraube übereinstimmt, monoaxial ausgerichtet.

Die Innenwandung der Tulpe weist eine Stufe und proximal hinter dieser eine Erweiterung auf, die sich in proximaler Richtung monoaxial verjüngt. Der Schraubenkopf reicht proximal bis zu dieser Stufe in die Tulpe. Das - monoaxiale - Andruckelement beginnt distal erst dort und weist an ihrem distalen Ende einen geringeren Durchmesser als die Erweiterung der Tulpe auf, so dass dort ein gewisses seitliches Spiel vorhanden ist und so das monoaxiale Andruckelement in der Tulpe leicht verkippt werden kann, um so eine durch die Spannvorrichtung erlaubte exzentrische Ausrichtung des Stabs nicht zu behindern, vielmehr aufzunehmen und zu ermöglichen.

Eine monoaxiale Pedikelschraube weist an ihrem proximalen Ende einen - monoaxialen - Pedikelschraubenkopf auf, der entsprechend geschwenkt werden kann. Der Schraubenkopf hat einen zylindrischen Mantel und eine flache proximale stirnseitige Vertiefung mit einem dem Umfangsrand des Andruckelements angepassten ebenfalls kegelringförmigen Rand.

Durch die Spannvorrichtung mit der Stabklemme können verkippte Anordnungen des Verbindungsstabs durch entsprechende Schwenkbewegung der Stabklemme und des Andruckelements eingestellt werden, was einerseits Belastungen der Befestigungsvorrichtung, andererseits die Belastung des Patienten zumindest erheblich reduziert; die Stabilität der Befestigungsvorrichtung, insbesondere deren Langzeitstabilität, sind hierdurch wesentlich vergrößert. Durch die Variabilität der Ausrichtung des Querstabs in der Vorrichtung wird die Stabilität des Aufbaus somit verbessert.

Weitere Vorteile und Merkmale der Erfindung ergeben sich aus den Ansprüchen und der nachfolgenden Beschreibung, in der Ausführungsbeispiele unter Bezugnahme auf die Zeichnungen im Einzelnen erläutert sind. Dabei zeigen:
- Fig. 1: eine erfindungsgemäße Befestigungsvorrichtung in einer Seitenansicht in polyaxialer Ausgestaltung;
- Fig. 2: die Vorrichtung der Fig. 1 in LängsSchnittdarstellung;
- Fig. 2a: die Vorrichtung der Fig. 1, 2 in einem Längsschnitt unter 90° zu dem der Fig. 2 mit eingestecktem Stab;
- Fig. 3: die Befestigungsvorrichtung der Fig. 1 bis 2a in einem vergrößerten Teil-Längsschnitt mit einer ersten Ausgestaltung einer Spannvorrichtung;
- Fig. 3a: die Befestigungsvorrichtung der Fig. 1 bis 3 in einem vergrößerten Teil-Längsschnitt unter 90° zu dem Schnitt der Fig. 3;
- Fig. 4: einen Längsschnitt durch die Spannvorrichtung der ersten Ausgestaltung mit zur Spannschraube geneigter Stabklemme;
- Fig. 4a: die Ausgestaltung der Fig. 4 in einem um 90° um die Längsachse A der Vorrichtung versetzten Längsschnitt;
- Fig. 4b: einen Stabklemmenadapter im Längsschnitt;
- Fig. 4c: eine distale Stirnseitenansicht des Stabklemmenadapters;
- Fig. 5: eine Befestigungsvorrichtung in einer zweiten ähnlich der der Fig. 1 bis 3a mit abgewandelter Ausbildung insbesondere der Spannvorrichtung;
- Fig. 5a: einen Längsschnitt durch die Vorrichtung der Fig. 5 entsprechend Fig. 2a mit einem ungleich 90° zur Längsachse der Vorrichtung ausgerichteten Querstab in einem zum Schnitt der Fig. 5 um 90° versetzten Längsschnitt;
- Fig. 5b: eine Spannschraube der zweiten Ausgestaltung der Fig. 5, 5a in Schnittdarstellung;
- Fig. 6: eine Stabklemme der zweiten Ausgestaltung der Fig. 5, 5a in Schnittdarstellung;
- Fig. 7: die Stabklemme von Fig. 6 in perspektivischer Ansicht;
- Fig. 8: eine Spannvorrichtung der zweiten Ausgestaltung in einem schematischen Schnitt;
- Fig. 9: die Spannvorrichtung von Fig. 8 in Schnittdarstellung um 90° axial gedreht;
- Fig. 10: die Spannvorrichtung von Fig. 8 mit gekippter Stabklemme;
- Fig. 11: die Spannvorrichtung von Fig. 10 in Schnittdarstellung und um 90° axial gedreht;
- Fig. 12: ein polyaxiales Andruckelement in Seitenansicht;
- Fig. 13: das Andruckelement der Fig. 12 in Schnittdarstellung und um 90° axial gedreht;
- Fig. 14: das Andruckelement der Fig. 12 und 13 in perspektivischer Darstellung;
- Fig. 15: eine polyaxiale Pedikelschraube in Seitenansicht;
- Fig. 16: die Pedikelschraube von Fig. 15 in Schnittdarstellung;
- Fig. 17: eine weitere Ausgestaltung einer Befestigungsvorrichtung in Seitenansicht;
- Fig. 18: die Befestigungsvorrichtung von Fig. 17 in Schnittdarstellung;
- Fig. 19: ein Teil der Befestigungsvorrichtung der Fig. 17 und 18 in vergrößertem Axialschnitt;
- Fig. 20: ein monoaxiales Andruckelement in Seitenansicht;
- Fig. 21: das Andruckelement von Fig. 20 in Schnittdarstellung und um 90° axial gedreht;
- Fig. 22: das Andruckelement der Fig. 20 und 21 in perspektivischer Ansicht;
- Fig. 23: eine monoaxiale Pedikelschraube in Seitenansicht mit teilweisem Vierfachgewinde; und
- Fig. 24: die Pedikelschraube der Fig. 23 im Längsschnitt.

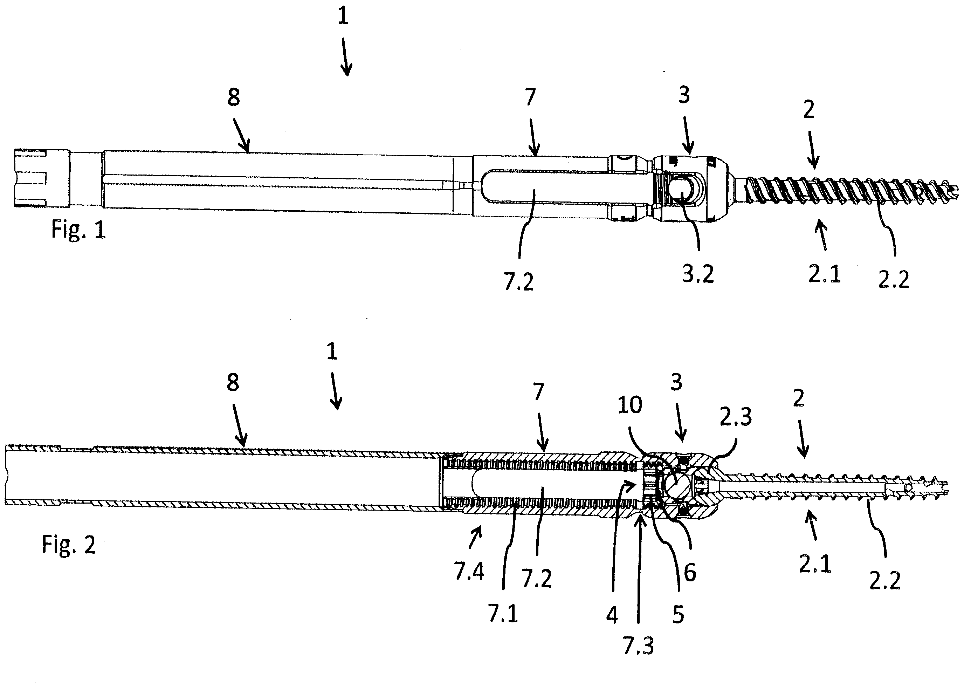

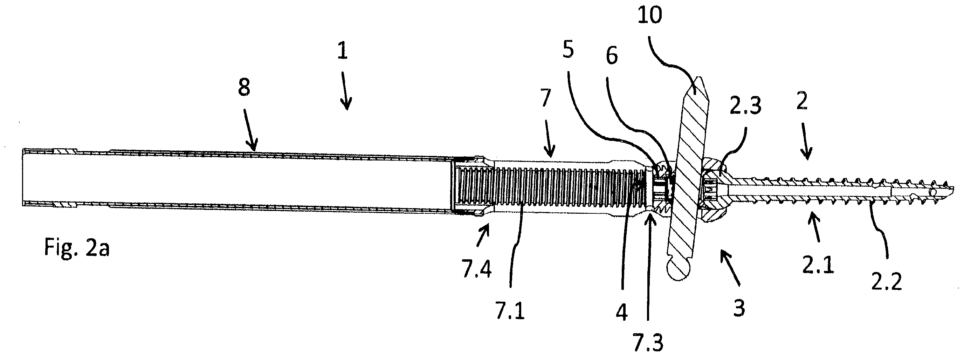

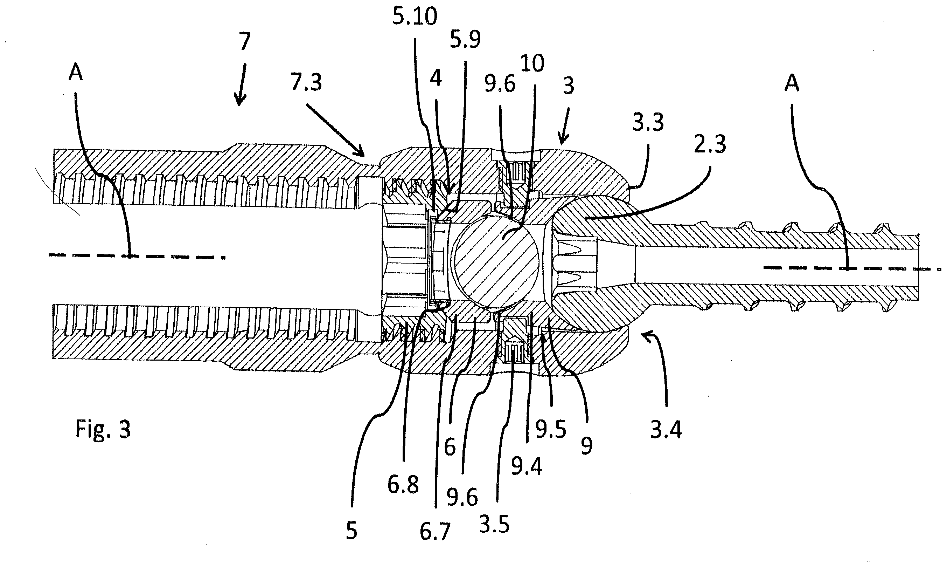

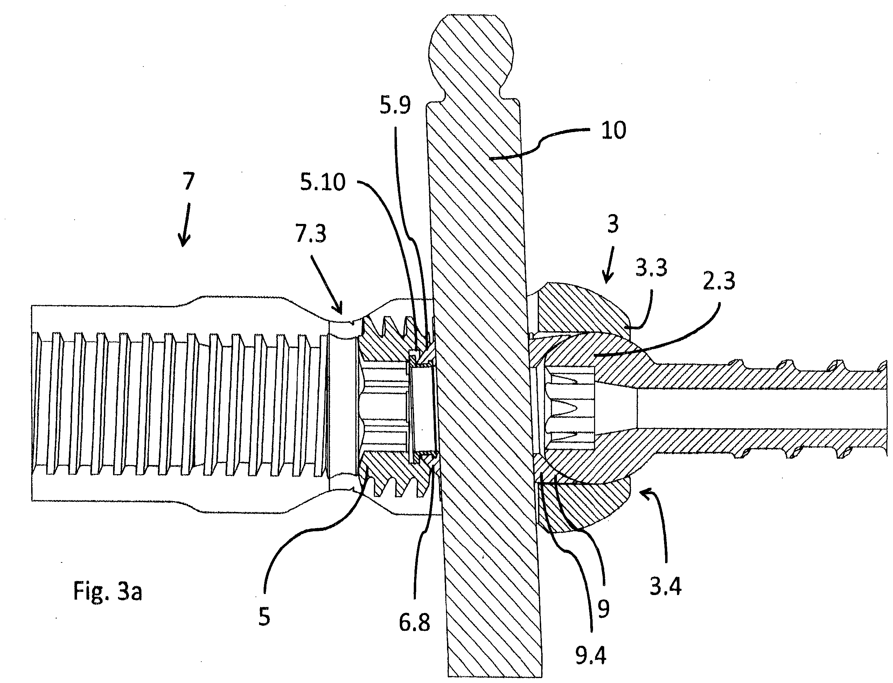

Fig. 1 zeigt eine Befestigungsvorrichtung 1 mit einer - polyaxialen - Pedikelschraube 2 und einer Tulpe 3. Die Vorrichtung 1 weist in ihrer Längsrichtung eine Hauptachse A auf. Sie hat weiter eine Spannvorrichtung 4 mit einer Spannschraube 5 und einer Stabklemme 6, die im Schnitt in der Fig. 2 und insbesondere auch Fig.3 dargestellt sind. Alle diese Teile sind kanüliert, d.h. sie weisen einen sich axial erstreckenden zentralen Hohlraum auf.

Im Folgenden bezeichnet "proximal" einen einem Operateur oder Benutzer - axial - zugewandten, distal einen diesem abgewandten und damit einem Patienten zugewandten bzw. in diesem liegenden Bereich der Vorrichtung 1 bei ihrem Einsatz.

Die Tulpe 3 hält die Pedikelschraube 2 und dient nach Einschrauben letzterer in einen Knochen, insbesondere einen Wirbel, zur Lagerung eines Stabs 10 (Fig. 2, 3) der sich insbesondere in Richtung der Wirbelsäule oder auch eines Langknochens oder in Richtung eines Flachknochens erstreckt und der auch durch weitere Tulpen mit Pedikelschrauben an einem anderen Knochen/Wirbel oder einer anderen Stelle z.B. eines Langknochens gehalten werden und die Knochen/Wirbel so fixieren kann. Die Tulpe 3 ist also eine Stablagerung und wird auch als solche bezeichnet.

An ihrem proximalen Ende ist die Tulpe 3 zunächst über eine Sollbruchstelle 7.3 mit einer Tulpenverlängerung 7 verbunden. Als Übergangsbereich zwischen der Tulpe 3 und der Tulpenverlängerung 7 weist die Sollbruchstelle 7.3 einen radial verringerten Querschnitt bzw. eine reduzierte Wandungsstärke auf. An einem proximalen Ende 7.4 der Tulpenverlängerung 7 weist diese ein Außengewinde (nicht dargestellt) zur Verbindung mit einem ein entsprechendes Innengewinde aufweisenden Verlängerungsschaft 8 auf.

Tulpe 3 und Tulpenverlängerung 7 sind wie der Verlängerungsschaft 8 kanüliert. Tulpe 3 und Tulpenverlängerung 7 weisen ein sich über beide erstreckendes Innengewinde 3.1, 7.1 auf. Weiter erstrecken sich im distalen Bereich der Tulpenverlängerung 7 und im proximalen Bereich der Tulpe 3 in der Wandung beider einander diametral gegenüberliegende Langlöcher 3.2, 7.2, die sich axial über den größten Teil der Tulpenverlängerung 7 und über etwa die Hälfte der Tulpe 3 erstrecken.

Die Pedikelschraube 2 hat einen Pedikelschraubenschaft 2.1 mit einem selbstschneidenden Pedikelgewinde 2.2. An dem proximalen Ende der Pedikelschraube 2 ist ein Pedikelschraubenkopf 2.3 ausgebildet, der von der Tulpe 3 umfasst ist (Fig. 1 bis 3).

Im distalen Bereich der Tulpe 3, in das der Pedikelschraubenkopf 2.3 eingebracht ist, ist ein Andruckelement 9 angeordnet, dessen Funktionsweise weiter unten beschrieben wird.

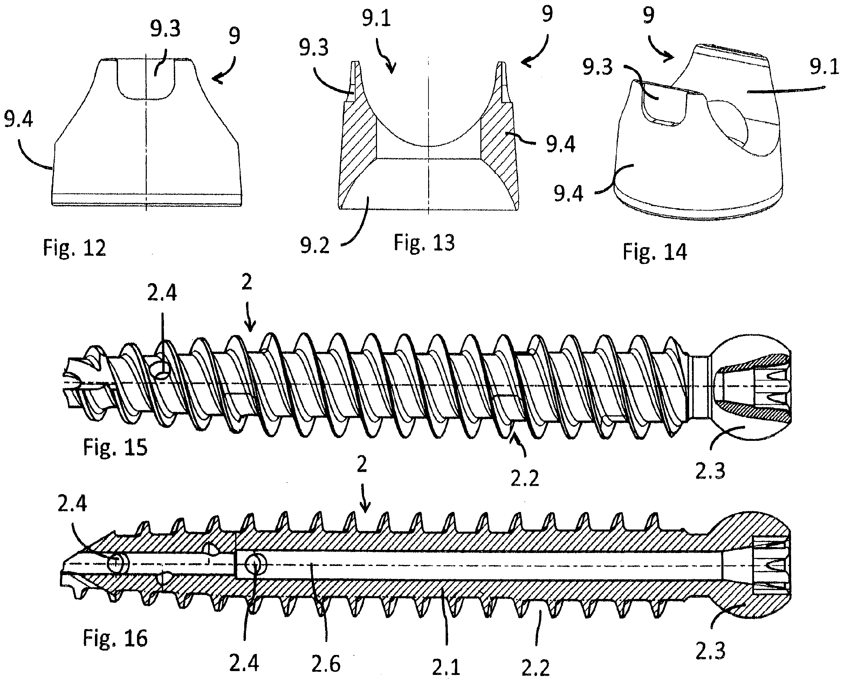

Die dargestellte Pedikelschraube 2 weist ein Zweifachgewinde auf, d.h. zwei Schraubengewindegänge S1, S2 (Fig. 15). Die Pedikelschraube 2 kann auch ein Einfach- oder (von Zwei- abweichenden) Mehrfachgewinde haben.

Der Pedikelschraubenschaft 2.1 ist mit Durchbrüchen 2.4 in einem Mantel 2.5 zu einem inneren Lumen 2.6 der Pedikelschraube 2 versehen. Das Lumen 2.6 durchdringt axial die gesamte Pedikelschraube 2. Durch das Lumen 2.6 und die Durchbrüche 2.4 können Flüssigkeiten und/oder insbesondere pastöse Massen, wie Reinigungsflüssigkeiten oder Knochenzement eingebracht werden.

Ein Außengewinde 5.2 einer Spannschraube 5 ist auf ein Innengewinde 3.1 der Tulpe 3 abgestimmt (weiter unten näher beschrieben).

In Fig. 2 (und auch zu den Fig. 2a bis 4a) ist ein in der Tulpe 3 eingespannter Stab 10 (im Schnitt) ersichtlich, der zwischen der ihm von der proximalen Seite umgreifenden Stabklemme 6, auf die die Spannschraube 5 wirkt, und dem distal angreifenden Andruckelement 9 eingespannt ist. Auf das Einspannen des Stabes 10 wird weiter unten eingegangen.

Die Fig. 3a zeigt einen Schnitt durch die erfindungsgemäße Vorrichtung 1 mit einem Stab 10 mit einem Winkel ungleich 90° zur Vorrichtungsachse - wie eine solche Ausrichtung durch die unter Bezug auf die im Weiteren beschriebene Ausgestaltung der Einzelelemente ermöglicht wird.

Die Spannschraube 5 (Fig. 3, 3a) hat eine axiale Symmetrieachse, die mit der Hauptachse A zusammenfällt. Am proximalen Ende der Spannschraube 5 ist eine mehrzählige nicht-zylindrische Vertiefung 5.1 vorgesehen, die einem Schraubenwerkzeug (Schraubenzieher, nicht dargestellt) einen formschlüssigen Eingriff zur Übertragung eines Drehmoments auf die Spannschraube 5 bietet. Die Vertiefung 5.1 kann als Vier- oder Mehrkant, insbesondere auch als Torx-Profil, ausgestaltet sein.

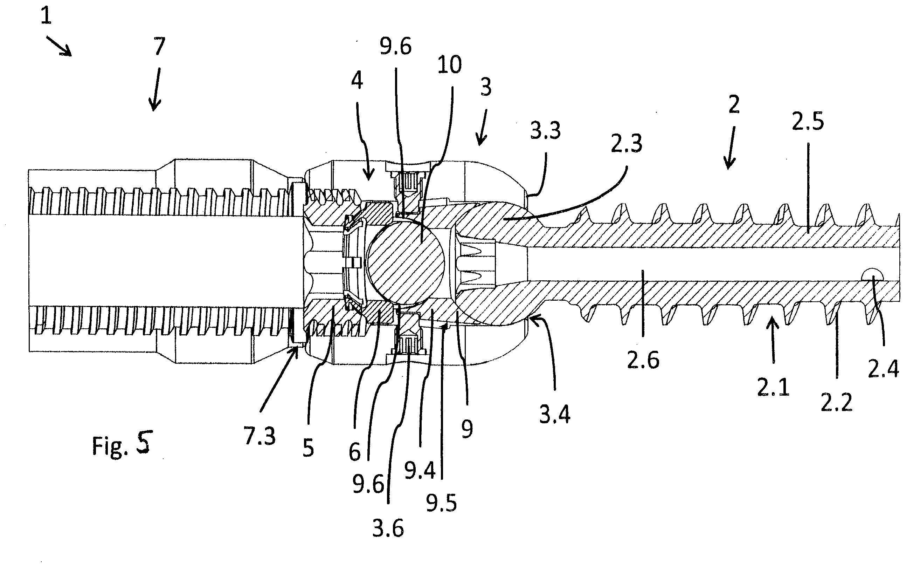

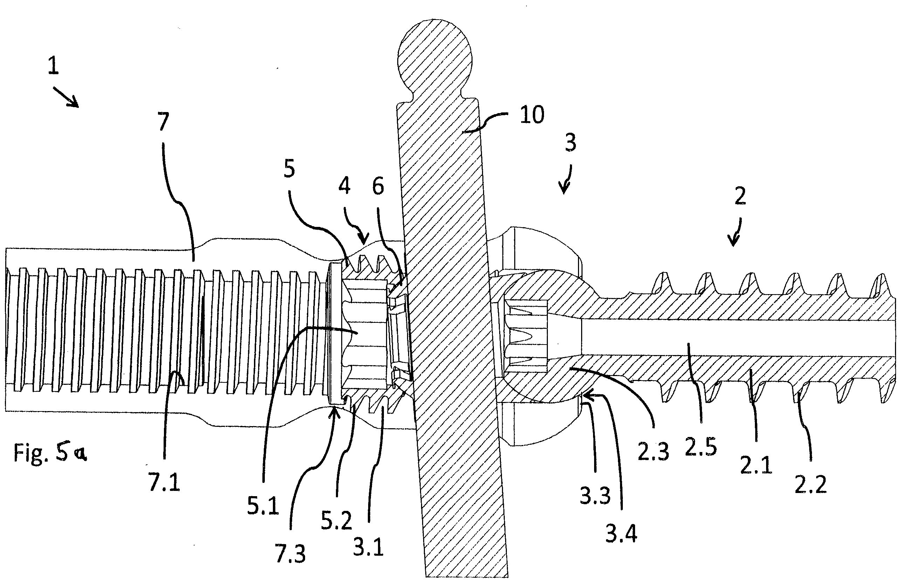

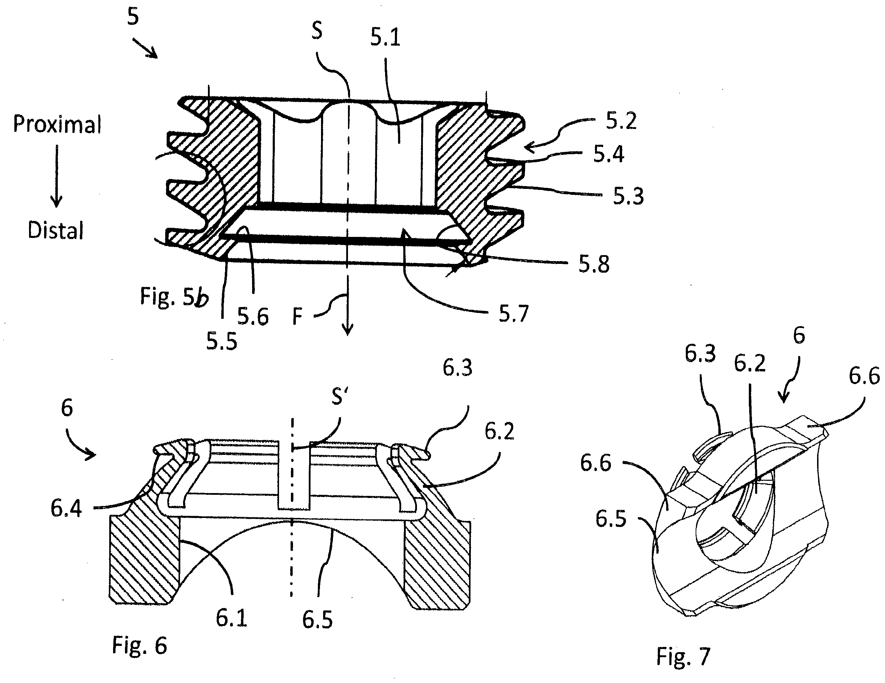

Die Außenseite der Spannschraube 5 ist mit dem asymmetrischen Spannschraubengewinde 5.2 versehen, wobei die Festschraubrichtung F distal gerichtet ist. In Fig. 5 ist das Spannschraubengewinde 5.2 als Sägengewinde ausgestaltet. Andere Gewindearten, insbesondere andere asymmetrische Gewindearten, sind ebenfalls möglich.

Bei dem Spannschraubengewinde 5.2 in Form eines speziellen Sägengewindes sind die Flanken 5.3, 5.4 gegenüber (achssenkrechten) Radialebenen gleichsinnig von innen nach außen in proximaler Richtung geneigt und zwar die distal gerichtete Flanke 5.3 eines Zahnkörpers hier um 30° und proximal gerichtete Flanke 5.4 der Schraubenwindung um 3°, so dass der Flankenwinkel (zwischen beiden Flanken) 27° beträgt. Ein solches Sägengewinde ist gegenüber entgegen der Festschraubrichtung wirkenden Kräften hoch belastbar.

An ihrem distalen Ende weist die Spannschraube 5 bei dieser ersten Ausgestaltung entsprechend insbesondere der Fig. 3 eine allgemein zur Achse A von außen distal und innen proximal schräg verlaufende ringförmige Stirnseite 5.9 in Form einer Kugelzone (als Mantel eine Kugelschicht) auf. Proximal hinter dieser ist eine radiale Ringnut 5.10 als Hinterschnitt ausgebildet. Eine Stabklemme 6 für den einzuspannenden Stab 10 weist an ihrem zur Spannschraube 6 hin gewandtes proximales Ende eine ebenfalls zur Achse A schräg verlaufende, der Stirnseite 5.9 entsprechende Stirnseite 6.7 auf. Auch die Stabklemme 6 ist an der Innenseite ihres Mantels mit einer radialen Ringnut 6.8 versehen. In die Ringnuten 5.10, 6.8 greifen - hier vier - nicht gleichmäßig über den Umfang angeordnete radial nach außen gerichtete Vorsprünge 6a.1 und 6a.2 eines ringförmigen Stabklemmenadapters (6a) ein, um so die Stabklemme 6 an der Spannschraube 5 gegen Herausfallen zu sichern. Der Stabklemmenadapter ist vorzugsweise mit der Stabklemme 6 verschweißt.

Fig. 5 zeigt eine Spannschraube 5 der zweiten Ausgestaltung der Spannvorrichtung 4 in einem axialen Längsschnitt. Soweit sie gleich wie die unter Bezug auf die Fig. 3, 3a beschriebene Spannschraube ist, wird auf die dortige Beschreibung verwiesen.

Die Spannschraube 5 weist proximal hinter ihrer distalen Stirnseite einen Ringvorsprung in Form eines sich von der distalen Stirnseite proximal erstreckenden Innenkonus auf, dem sich über eine Hinterschneidung 5.6 proximal ein Lumen 5.7 der Spannschraube 5 in proximaler Richtung verjüngender Innenkonus 5.8 anschließt, der axial am distalen Endbereich der Vertiefung 5.1 der Spannschraube 5 endet und dort radial einen größeren Durchmesser als die Vertiefung 5.1 hat. Die Stabklemme 6 ist ebenfalls ringförmig mit einem Lumen ausgebildet (Fig. 6) und an ihrer distalen Stirnseite mit einer Bohrung 6.1 versehen, die sich axial über etwa die Hälfte der Höhe der Stabklemme 6 erstreckt sowie radial einen konstanten Querschnitt hat. Am proximalen Ende der Bohrung 6.1 weist die Stabklemme 6 eine Reihe von in Umfangsrichtung nebeneinander angeordneten und in proximaler Richtung und radial nach innen gerichteten elastischen Fingern 6.2 auf, die an ihrem Endbereich radial nach außen weisende Nasen 6.3 haben, die Hinterschneidungen 6.4 bilden. Die axiale Symmetrieachse S' der Stabklemme 6 fällt mit der Symmetrieachse S der Spannschraube 5 zusammen. Auf das Zusammenwirken der Spannschraube 5 mit der Stabklemme 6 wird weiter unten eingegangen.

Die distale Stirnseite der Stabklemme 6 hat eine teilzylindrische Klemmaussparung 6.5 zur Aufnahme des Querstabs 10 (Fig. 7). Die Stabklemme 6 weist in Richtung der einen Symmetrieachse der teilzylindrischen Ausnehmung 6.5 beidseits Ansätze 6.6 auf.

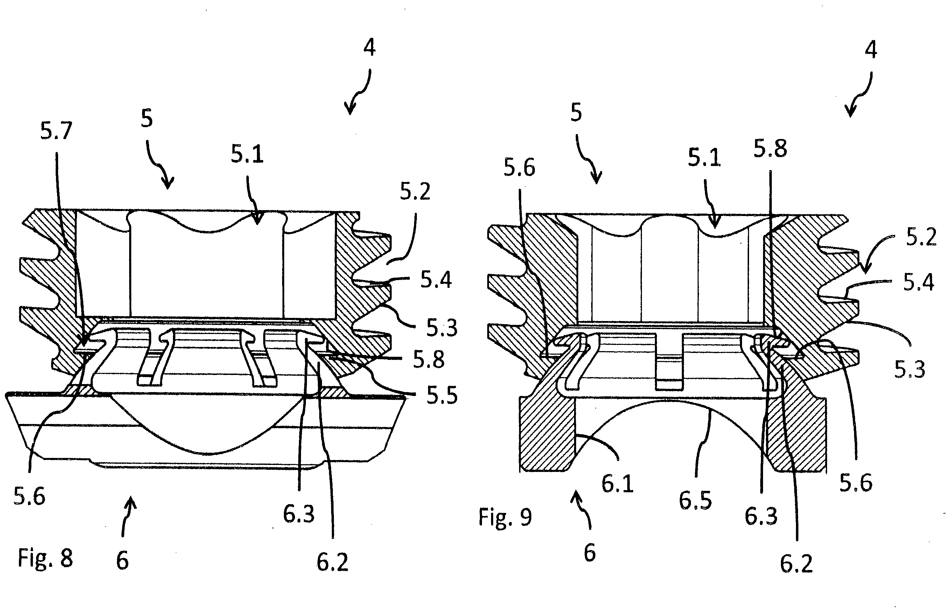

Fig. 8 stellt die Spannvorrichtung 4 mit zusammengesteckter Spannschraube 5 und Stabklemme 6 dar. Hierzu wurden die Spannschraube 5 und die Stabklemme 6 axial aufeinander zubewegt, so dass die Nasen 6.3 der Stabklemme 15 zunächst in Kontakt mit dem konusförmigen Ringvorsprung 5.5 der Spannschraube 5 kommen. Bei weiterer Axialbewegung werden die Finger 6.2 durch die Konusflanke des Ringvorsprungs 5.5 radial nach innen gedrückt, wodurch eine weitere relative Axialbewegung erlaubt wird. Gelangen die Nasen 6.3 der Finger 6.2 zur Hinterschneidung 5.6 jenseits des Ringvorsprungs 5.5, so rasten die Nasen 6.3 mit Hinterschneidungen 6.4 in der durch den Ringvorsprung 5.5 gebildeten Hinterschneidung 5.6 der Spannschraube 5 ein. Dadurch ist die Spannschraube 5 mit der Stabklemme 6 nicht verlierbar verbunden. Fig. 8 und 9 zeigen um 90° zueinander verdrehte Schnittdarstellungen.

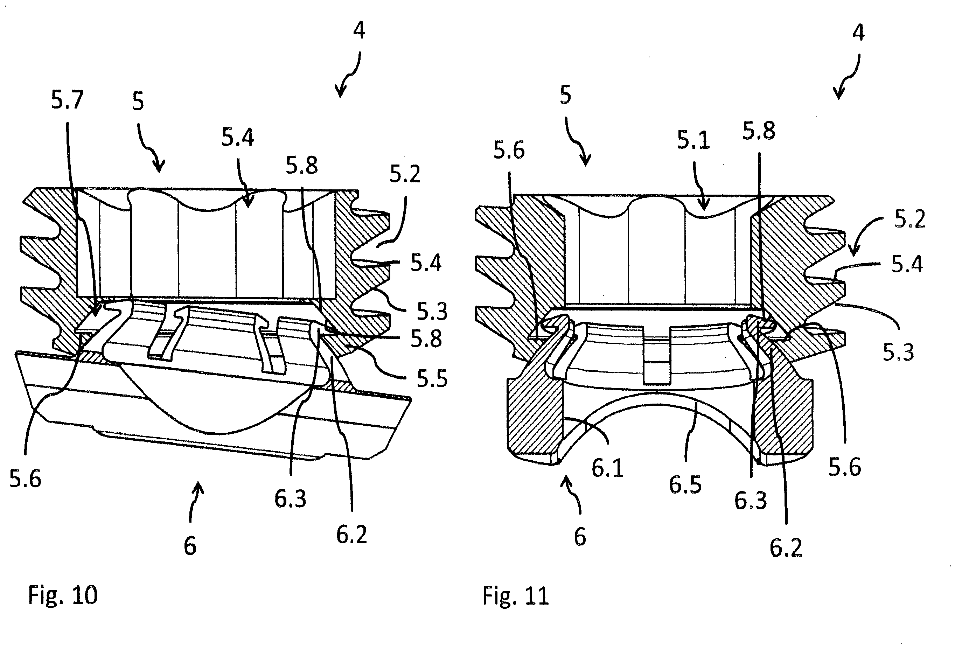

Aufgrund der Dimensionierung des Innenkonus 5.8 der Spannschraube 5 ist eine Kippbewegung der Stabklemme 6 in der Spannschraube 5 möglich, wie dies in den Fig. 10 und 11 mit einem Kippwinkel von etwa 6° zur Symmetrieachse S der Spannschraube 5 dargestellt ist. Durch die Ausgestaltung des sich proximal verjüngenden Innenkonus 5.8 befinden sich auch in gekippter Stellung der Stabklemme 6 deren Nasen 6.3 in Kontakt mit der Innenwand des Innenkonus 5.8. Auf diese Weise ist die radiale Festlegung der Stabklemme 6 in axial ausgerichteter und in gekippter Stellung gewährleistet. Fig. 11 zeigt einen um 90° verdrehten Axialschnitt der Spannvorrichtung 4 der Fig. 10.

Die Fig. 1 bis 4 zeigen - wie gesagt - eine polyaxiale Ausgestaltung der Befestigungsvorrichtung 1. Diese weist weiter ein polyaxiales Andruckelement 9 (Fig. 12-14) und diese beschriebene Pedikelschraube 2 mit einem kugelförmigen Schraubenkopf 2.3 (Fig. 15, 16) auf.

Das polyaxiale Andruckelement 9 (Fig. 12-14) weist auf seiner proximalen Seite eine teilzylindrische Ausnehmung 9.1 zum teilweisen Umfassen bzw. Aufnehmen des Stabs 10 auf, während eine weitere halbkugel- oder kalottenförmige Ausnehmung 9.2 an der distalen Stirnseite des Andruckelements 9 vorgesehen ist, um den kugelförmigen Schraubenkopf 2.3 der Pedikelschraube 2 zu erfassen.

Das gegenüber dem Stab 10 distale Andruckelement 9 hat einen sich von seinem distalen Ende zum proximalen Ende hin verjüngenden Mantel 9.4, so dass zwischen diesem und der Innenwandung der Tulpe 3 ein Spalt 9.5 gebildet ist (insbesondere Fig. 3).

Hierdurch ist ein Verkippen des Andruckelements 9 innerhalb der Tulpe 3 gegenüber einer axial ausgerichteten zentrischen Stellung möglich. So kann das Andruckelement 9 eine entsprechend durch die beschriebene Spannvorrichtung 4 dem Stab 10 ermöglichte gekippte Ausrichtung einnehmen und in dieser festgespannt werden und zwar in sowohl mit einer gegenüber der Ausrichtung der einander gegenüberliegender Langlöcher 3.2 in den Wandungen der Tulpe 3 angular versetzten, also auch in einer gegenüber der Achssenkrechten gekippten Ausrichtungen. Die Spannvorrichtung 4 ermöglicht derart solche gekippten Ausrichtungen des Stabs 10 und behindert diese nicht.

Die teilzylindrische Ausnehmung 9.1 weist einen größeren Krümmungsradius als der in der Fig. 3 dargestellte Stab 10 auf. Dadurch ergibt sich bei der proximalen Öffnung des Andruckelements 9 etwa auf halber Höhe des Stabs 10 ein Spiel 9.6 senkrecht zur Erstreckung des Stabs 10. Hierdurch können Stäbe 10 mit unterschiedlichem Durchmesser in der gleichen Befestigungsvorrichtung 1 eingesetzt werden. Wenn der dargestellte Stab beispielsweise einen Durchmesser von 5,5 mm hat, wie er bei den meisten Personen eingesetzt und üblich ist und die zylindrische Ausnehmung einen Durchmesser von 6 mm hat, so kann auch ein Stab mit 6 mm Durchmesser beispielsweise bei kräftigen, insbesondere jungen Personen, eingesetzt werden, bei denen oft ein Stab mit 5,5 mm Durchmesser nicht die notwendige Festigkeit hat.

An der radialen Außenseite des Andruckelements 9 sind im distalen Bereich zwei gegenüber liegende vertiefte Andruckflächen 9.3 für radial von außen durch die Tulpe 3 einschraubbare Querschrauben 3.5 zum Fixieren des Andruckelements 9 innerhalb der Tulpe 3 vorgesehen.

Die polyaxiale Pedikelschraube 2 selbst ist in den Fig. 15 und 16 gezeigt und weist - wie schon oben gesagt - den kugelförmigen Pedikelschraubenkopf 2.3 auf. Der Pedikelschraubenkopf 2.3 ist in der kalottenförmigen Ausnehmung 9.2 am distalen Bereich des polyaxialen Andruckelements 9 in allen Richtungen schwenkbar. Der Schraubenkopf 2.3 und damit die Pedikelschraube 2 wird mittels des Andruckelements 9 - unverlierbar - in der Tulpe 3 gehalten, da diese an ihrem distalen Ende 3.3 eine gegenüber dem Durchmesser des Schraubenkopfes verengte Öffnung aufweist (Fig. 1-4).

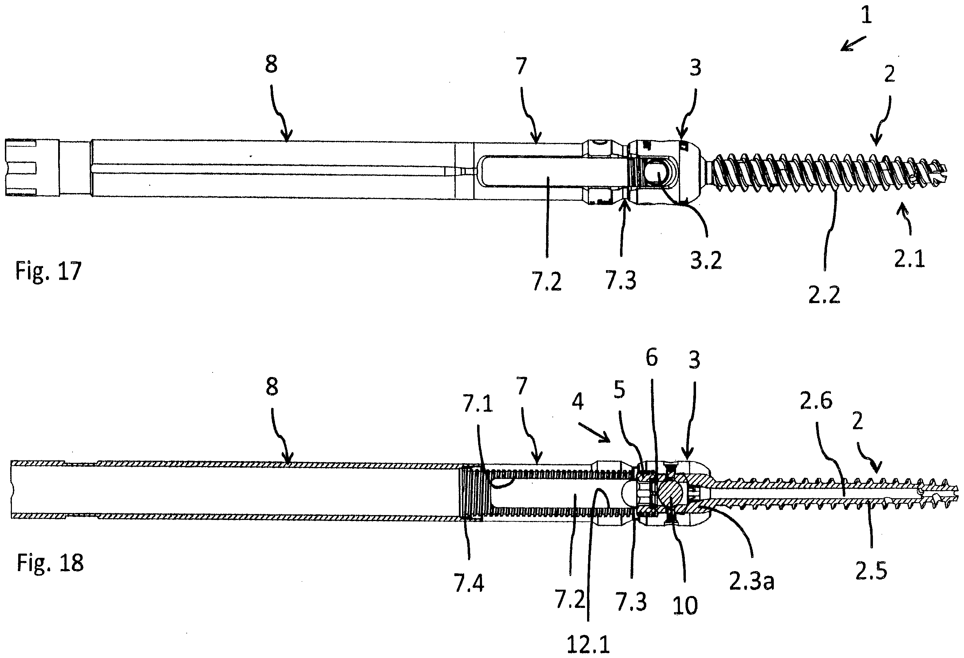

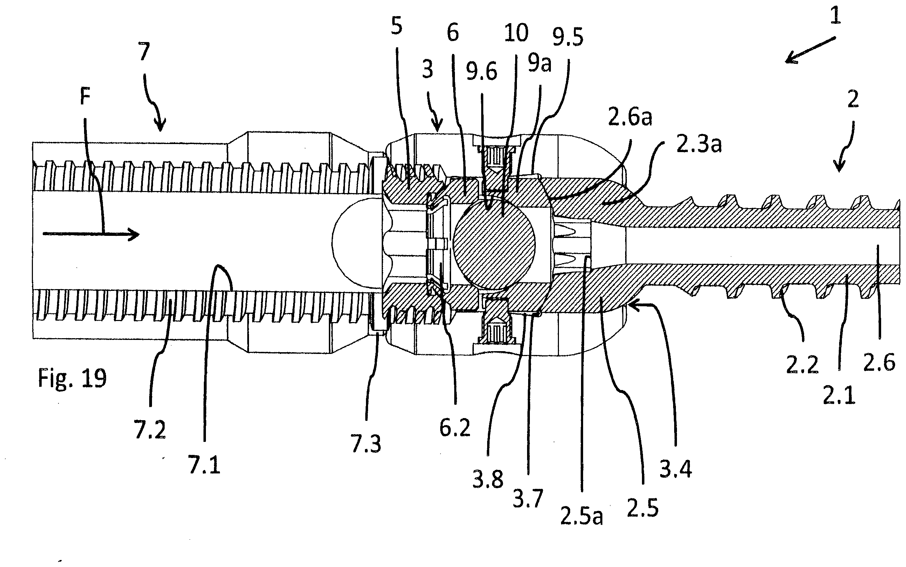

Bei einer monoaxialen Vorrichtung der Fig. 17 ist insbesondere die Spannvorrichtung 4 mit Spannschraube 5 und Stabklemme 6 die gleiche wie bei der vorher beschriebenen polyaxialen Befestigungsvorrichtung. Unterschiedlich sind lediglich ein - monoaxialer - Pedikelschraubenkopf 2.3a der Pedikelschraube 2 und ein - monoaxiales - Andruckelement 9a, die in vergrößerter Darstellung in den Fig. 19-24 dargestellt sind.

Die Fig. 17 bis 19 zeigen eine monoaxiale Befestigungsvorrichtung 1 in Seitenansicht (Fig. 17) bzw. Längsschnitt (Fig. 18, 19). Gleiche Teile und Merkmale sind grundsätzlich mit gleichen Bezugszeichen bezeichnet wie bei der polyaxialen Ausgestaltung der Fig. 1 bis 16, worauf auch Bezug genommen wird. Im Folgenden sind hier die unterschiedlichen Ausprägungen erläutert.

Bei der Spannvorrichtung 1 der Fig. 17 bis 19 ist distal des eingespannten Querstabs 10 die Tulpe 3 mit dem monoaxialen Andruckelement 9 ersichtlich. Das Andruckelement 9 hält den Pedikelschraubenkopf 2.3a, während die Pedikelschraube 2 selbst in einem Wirbelkörper eingeschraubt und fixiert ist (nicht dargestellt). Proximal des Querstabs 10 befindet sich in der Tulpe 3 mit Hilfe eines Drehwerkzeugs (nicht dargestellt) die entlang des Innengewindes 7.1 der Tulpenverlängerung 7 in Festschraubrichtung F eingeschraubte Spannschraube 5, die auf die Stabklemme 6 wirkt. Diese nimmt - hier - eine verkippte Stellung ein und kann so den Querstab 10 senkrecht in einer polyaxialen Erstreckungsrichtung halten.

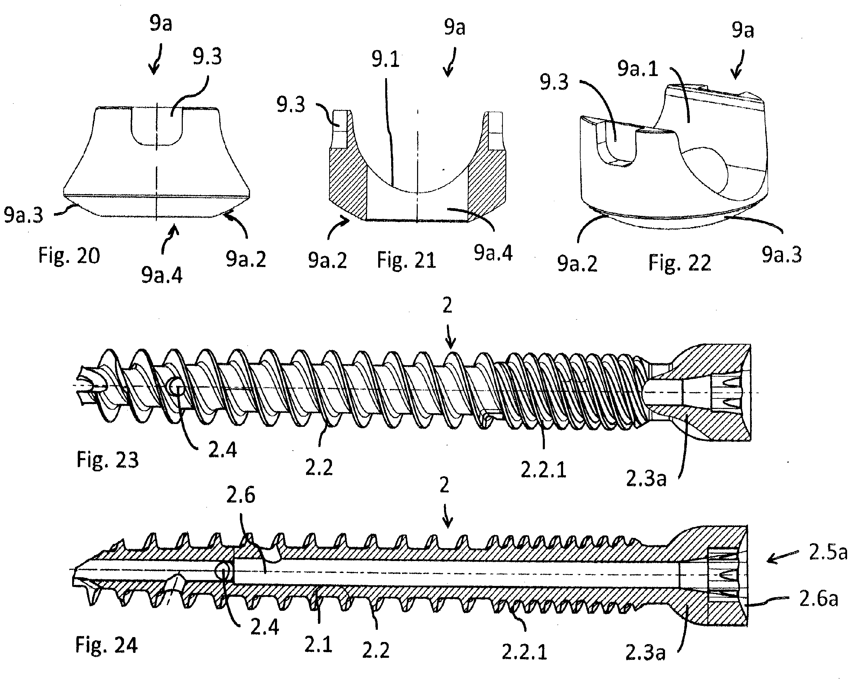

Das monoaxiale Andruckelement 9a weist ebenso wie das polyaxiale Andruckelement 9 eine proximale teilzylindrische Ausnehmung 9.1 zur teilweisen Aufnahme des Stabs 10, abweichend aber einen dem Schraubenkopf 2.3a der Pedikelschraube 2, zugewandte flache kegelstumpfförmige distale Stirnseite 9a.2 mit einem kegelringförmigen Umfangsrand 9a.3 auf, der einen zentralen Durchbruch 9a.4 umgibt (Fig. 20-22).

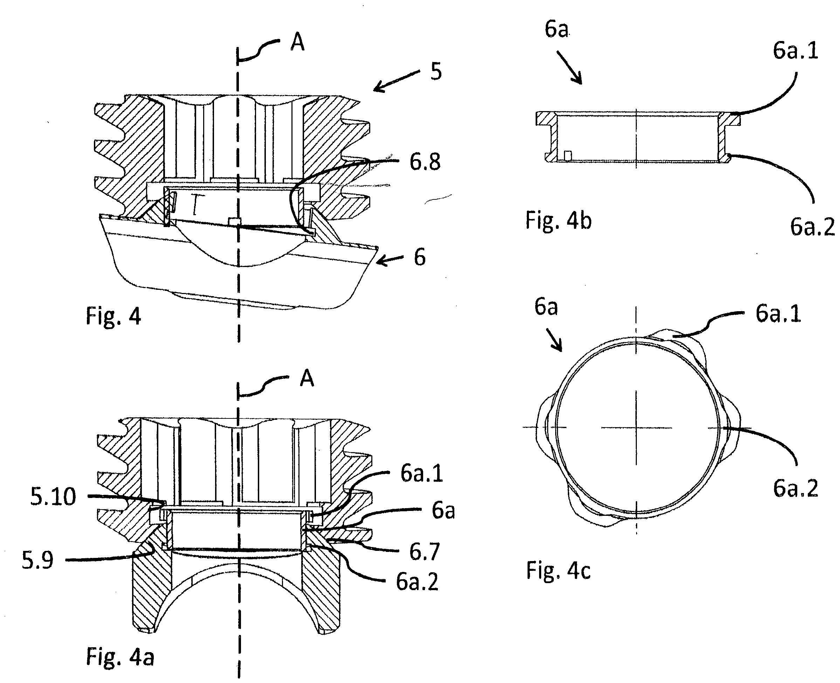

Die Innenwandung der Tulpe 3 weist eine Stufe 3.7 und proximal hinter dieser eine Erweiterung 3.8 auf, die sich in proximaler Richtung monoaxial verjüngt. Bei der monoaxialen Ausgestaltung der Fig. 17 reicht der Schraubenkopf 9a proximal bis zu dieser Stufe 3.7 in die Tulpe 3. Das - monoaxiale - Andruckelement 9a beginnt distal erst dort und weist an seinem distalen Ende einen geringeren Durchmesser als die Erweiterung 3.8 auf, so dass dort ein gewisses seitliches Spiel vorhanden ist und so auch das monoaxiale Andruckelement 9a in der Tulpe 3 leicht verkippt werden kann, um so eine durch die Spannvorrichtung 4 erlaubte exzentrische Ausrichtung des Stabs, wie dies in Fig. 4, für die polyaxiale Vorrichtung 1 dargestellt ist, nicht zu behindern, vielmehr aufzunehmen und zu ermöglichen.

Die Fig. 22 zeigt das monoaxiale Andruckelement 9a in einer perspektivischen Ansicht; die gegenüber der Querrahmung des Stabs 10 erweiterte Ausnehmung 9.1 erlaubt eine exzentrische Fixierung des Stabs 10.

Die Pedikelschraube 2, wie insbesondere in den Fig. 18, 23 und 24 dargestellt ist, weist an ihrem proximalen Ende einen - monoaxialen Pedikelschraubenkopf 2.3a auf, der entsprechend geschwenkt werden kann. Der Schraubenkopf 2.3a weist einen zylindrischen Mantel 2.5 und eine flache proximale stirnseitige Vertiefung 2.5a auf, der einen dem Umfangsrand 9a.3 angepassten ebenfalls kegelringförmigen Rand 2.6a hat (Fig. 19, 24).

Während die bis zu der Fig. 22 dargestellte Pedikelschrauben 2 über die gesamte Länge des Schraubenschaftes 2.1 ein Zweifachgewinde haben, weist die in den Fig. 23, 24 dargestellte Pedikelschraube 2 im proximalen Bereich des Schraubenschaftes 2.1 über um etwa ein Viertel der Länge des Gewindes bzw. Schraubenschaftes 2.1 ein Vierfachgewinde 2.2.1 auf. Eine solche Ausgestaltung kann auch bei einer polyaxialen Pedikelschraube gemäß den Fig. 1 bis 4, 15, 16 vorgesehen sein.

## Patentansprüche

1. Vorrichtung (1) zum Befestigen eines Stabs an einem Knochen, mit einer Pedikelschraube (21), einer diese haltenden Tulpe (3), einer Spannschraube (5), eine zur Spannschraube (5) schwenkbare Stabklemme (6), ein der Stabklemme (6) gegenüber in der Tulpe (3) gelagertes Andruckelement (9, 9a), **dadurch gekennzeichnet, dass** das Andruckelement (9, 9a) einen sich von seinem distalen Ende zum proximalen Ende hin verjüngenden Mantel hat, so dass zwischen diesem und der Innenwandung der Tulpe (3) ein Spalt gebildet ist, der ein Verkippen des Andruckelements (9, 9a) innerhalb der Tulpe (3) gegenüber einer axial ausgebildeten zentrischen Stellung ermöglicht.

2. Vorrichtung nach Anspruch 1, **dadurch gekennzeichnet, dass** die Stabklemme (6) relativ zur Spannschraube (5) über einander zugewandte schräg zu einer Hauptachse (A) ausgerichtete beidseitige Kugelzonen (6.7, 5.9) verschwenkbar ist.

3. Vorrichtung nach Anspruch 1 oder 2, **dadurch gekennzeichnet, dass** Spannschraube (5) und Stabklemme (6) einander hintergreifende Hinterschneidungen (5.6, 6.4) aufweisen, wobei insbesondere die Hinterschneidung (5.6) der Spannschraube (5) durch einen Ringvorsprung (5.5) gebildet ist.

4. Vorrichtung nach Anspruch 3, **dadurch gekennzeichnet, dass** die Hinterschneidungen (6.4) der Stabklemme (6) durch über den Umfang angeordnete elastische Finger (6.2) mit Nasen (6.3) gebildet sind.

5. Vorrichtung nach einem der Ansprüche 3 oder 4, **dadurch gekennzeichnet, dass** die Spannschraube (5) einen sich in proximaler Richtung von der Hinterschneidung (5.6) fort verjüngenden Innenkonus (5.8) aufweist.

6. Vorrichtung nach Anspruch 1 oder 2, **gekennzeichnet durch** einen Spannschraube (5) und Stabklemme (6) verbindenden Stabklemmenadapter (6a), wobei insbesondere die Stabklemme (6) und Stabklemmenadapter (6a) miteinander verschweißt sind.

7. Vorrichtung nach Anspruch 6, **dadurch gekennzeichnet, dass** der Stabklemmenadapter (6a) ein Ringteil mit proximalen nach außen radial gerichtete Vorsprüngen ist, die in radiale Einschnitte in der Spannschraube (5) unter Spiel eingreifen, wobei insbesondere distale, ebenfalls radial nach außen gerichtete Vorsprünge vorgegeben sind, die in radiale Einschnitte der Stabklemme (6) eingreifen, wobei insbesondere die Stabklemme (6) und Stabklemmenadapter (6a) miteinander verschweißt sind.

8. Vorrichtung nach einem der vorangehenden Ansprüche, **dadurch gekennzeichnet, dass** die Stabklemme (6) auf ihrer der Spannschraube (5) abgewandten Seite eine teilkreisförmige Klemmausnehmung (6.5) aufweist und/oder, dass die Spannschraube (5) ein asymmetrisches Spannschraubengewinde (5.2) aufweist.

9. Vorrichtung nach Anspruch 8, **dadurch gekennzeichnet, dass** eine in Festschraubrichtung (F) proximal gerichtete Flanke (5.4) des Gewindes (5.2) der Spannschraube (5) sich unter einem Winkel von kleiner gleich 5°, insbesondere kleiner gleich 3°, zu einer Radialebene zur Symmetrieachse (S) der Spannschraube (5) erstreckt.

10. Vorrichtung nach Anspruch 8, **dadurch gekennzeichnet, dass** eine in Festschraubrichtung (F) distal gerichtete Flanke (5.3) des Gewindes (5.2) der Spannschraube (5) sich unter einem von 90° abweichenden Winkel zur Symmetrieachse (S) der Spannschraube (5), vorzugsweise um 30°, zu einer Radialebene erstreckt.

11. Vorrichtung nach einem der vorangehenden Ansprüche, **dadurch gekennzeichnet, dass** die Spannschraube (5) auf ihrer der Stabklemme (6) abgewandten Seite eine mehrzählige oder nicht-zylindrische Vertiefung (5.1) aufweist.

12. Vorrichtung nach einem der vorangehenden Ansprüche, **dadurch gekennzeichnet, dass** das Andruckelement (9, 9a) kippbar in der Tulpe (3) gelagert ist.

13. Vorrichtung nach Anspruch 12, **dadurch gekennzeichnet, dass** der Durchmesser des Andruckelements (9, 9a) an mindestens einer seiner Stirnseiten gegenüber dem Innendurchmesser der Tulpe (3) reduziert ist, so dass das Andruckelement (9, 9a) innerhalb der Tulpe (3) kippbar ist.

14. Vorrichtung nach einem der Ansprüche 12 oder 13, **dadurch gekennzeichnet, dass** das Andruckelement (9) der Tulpe (3) sich von seiner distalen zu seiner proximalen Stirnseite verjüngt, so dass proximal ein radiales Spiel (9.6) zwischen Innenwand der Tulpe (3) und dem Mantel (9.4) des Andruckelements (9) gegeben ist oder dass die Innenwandung der Tulpe (3) sich von der Höhe der proximalen zur Höhe der distalen Stirnseite des Andruckelements (9a) unter Gewährleistung eines radialen Spiels (9.6) zwischen Andruckelement (9a) und Tulpe (3) auf Höhe der distalen Stirnseite des Andruckelements (9a) erweitert.

15. Vorrichtung nach einem der vorangehenden Ansprüche, **dadurch gekennzeichnet, dass** der Schraubenschaft (2.1) der Pedikelschraube (2) ein Zweifachgewinde(2.2) aufweist, wobei insbesondere ein proximaler Bereich des Schraubenschaftes (2.1) als Vierfachgewinde (2.2.1) ausgebildet ist, vorzugsweise über eine Länge von ein Viertel des Pedikelschraubenschaftes (2.1).

## Claims

1. Device (1) for fixating a rod on a bone, with a pedicle screw (2), with a tulip (3) holding the pedicle screw (2), with a tightening screw (5), a rod clamp (6) pivotable to the tightening screw (5), a pressing element (9, 9a), which is mounted opposite the rod clamp (6) in the tulip (3), **characterized in that** the pressing element (9, 9a) has a jacket which tapers from its distal end face to its proximal end face, so that there is a radial clearance (9.6) between it and the inner wall of the tulip (3) which enables a tilting of the pressing element (9, 9a) in the tulip (2) relative to an axially centered position.

2. Device in accordance with claim 1, **characterized in that** the rod clamp (6) is pivotable to the tightening screw (5) by means of bilateral spherical zones (6.7, 5.9) facing one another and oriented obliquely to a principal axis (A).

3. Device in accordance with claim 1 or 2, **characterized in that** the tightening screw (5) and the rod clamp (6) have undercuts (5.6, 6.4) extending behind one another wherein in particular the undercut (5.6) of the tightening screw (5) is formed by a ring projection (5.5)

4. Device in accordance with claim 3, **characterized in that** the undercuts (6.4) of the rod clamp (6) are formed by elastic fingers (6.2) with bosses (6.3), which are arranged over the circumference.

5. Device in accordance with one of the claims 3 or 4, **characterized in that** the tightening screw (5) has an inner cone (5.8) tapering in the proximal direction away from the undercut (5.6).

6. Device in accordance with claim 1 or 2, **characterized by** a rod clamp adapter (6a) connecting the tightening screw (5) and the rod clamp (6) wherein in particular the rod clamp (6) and the rod clamp adapter (6a) are welded together.

7. Device in accordance with claim 6, **characterized in that** the rod clamp adapter (6a) is a ring part with proximally radially outwardly directed projections, which mesh with radial incisions in the tightening screw (5) with a clearance, wherein especially distally, likewise radially outwardly directed projections are predefined, which mesh with radial incisions of the rod clamp (6) wherein in particular the rod clamp (6) and the rod clamp adapter (6a) are welded together..

8. Device in accordance with one of the above claims, **characterized in that** the rod clamp (6) has a partially circular clamping recess (6.5) on its side facing away from the tightening screw (5) and/or that the tightening screw (5) has an asymmetric tightening screw thread (5.2).

9. Device in accordance with claim 8, **characterized in that** a flank (5.4) of the thread (5.2) of the tightening screw (5), which flank is directed proximally in relation to the screwdown direction (F), extends at an angle smaller than or equal to 5°, especially smaller than or equal to 3°, to a radial plane to the symmetry axis (S) of the tightening screw (5).

10. Device in accordance with claim 8, **characterized in that** a flank (5.3) of the thread (5.2) of the tightening screw (5), which flank is directed distally in the screwdown direction (F), extends at an angle different from 90° to the symmetry axis (S) of the tightening screw (5), preferably by 30°, to a radial plane.

11. Device in accordance with one of the above claims, **characterized in that** the tightening screw (5) has a plurality of depressions or a non-cylindrical depression (5.1) on its side facing away from the rod clamp (6).

12. Device in accordance with one of the above claims, **characterized in that** the pressing element (9, 9a) is mounted tiltably in the tulip (3).

13. Device in accordance with claim 12, **characterized in that** the diameter of the pressing element (9, 9a) is reduced on at least one of its end faces compared to the internal diameter of the tulip (3), so that the pressing element (9, 9a) in the tulip (3) is tiltable.

14. Device in accordance with one of the claims 12 or 13, **characterized in that** the pressing element (9) of the tulip (3) tapers from its distal end face to its proximal end face, so that there is a radial clearance (9.6) proximally between the inner wall of the tulip (3) and the jacket (9.4) of the pressing element (9) or that the inner wall of the tulip (3) expands from the level of the proximal end face of the pressing element (9a) to the level of the distal end face of the pressing element (9a) while a radial clearance (9.6) is guaranteed between the pressing element (9a) and the tulip (3) at the level of the distal end face of the pressing element (9a).

15. Device in accordance with one of the above claims, **characterized in that** the screw shaft (2.1) of the pedicle screw (2) has a double thread (2.2), wherein especially a proximal area of the screw shaft (2.1) is configured as a quadruple thread (2.2.1), preferably over a length of one fourth of the pedicle screw shaft (2.1).

## Revendications

1. Dispositif (1) de fixation d'une tige sur un os, avec une vis pédiculaire (2), une tulipe (3) la maintenant, une vis de serrage (5), un serre-tige (6) pouvant pivoter par rapport à la vis de serrage (5), un élément de compression (9, 9a) disposé dans la tulipe (3) en vis-à-vis par rapport au serre-tige (6), **caractérisé en ce que** l'élément de compression (9, 9a) a une enveloppe se rétrécissant depuis son extrémité distale vers son extrémité proximale, de sorte qu'entre celle-ci et la paroi intérieure de la tulipe (3), une fente soit formée qui permette un basculement de l'élément de compression (9, 9a) à l'intérieur de la tulipe (3) par rapport à une position centrale réalisée dans le plan axial.

2. Dispositif selon la revendication 1, **caractérisé en ce que** le serre-tige (6) peut être pivoté par rapport à la vis de serrage (5) via des zones sphériques (6.7, 5.9) bilatérales orientées de façon oblique par rapport à un axe principal (A) et orientées l'une vers l'autre.

3. Dispositif selon la revendication 1 ou 2, **caractérisé en ce que** la vis de serrage (5) et le serre-tige (6) comportent des détourés arrière (5.6, 6.4) s'imbriquant l'un l'autre par l'arrière, le détouré arrière (5.6) de la vis de serrage (5) étant notamment formé par une saillie annulaire (5.5).

4. Dispositif selon la revendication 3, **caractérisé en ce que** les détourés arrière (6.4) du serre-tige (6) sont formés par des doigts (6.2) élastiques disposés sur toute la périphérie, avec des becs (6.3).

5. Dispositif selon l'une quelconque des revendications 3 ou 4, **caractérisé en ce que** les vis de serrage (5) comportent un cône intérieur (5.8) se rétrécissant dans la direction proximale à partir du détouré arrière (5.6).

6. Dispositif selon la revendication 1 ou 2, **caractérisé par** la présence d'un adaptateur de serre-tige (6a) reliant la vis de serrage (5) et le serre-tige (6), le serre-tige (6) et l'adaptateur de serre-tige (6a) étant notamment soudés entre eux.

7. Dispositif selon la revendication 6, **caractérisé en ce que** l'adaptateur de serre-tige (6a) est une partie annulaire avec des saillies proximales orientées vers l'extérieur dans le plan proximal qui s'engrènent avec du jeu dans les entailles radiales dans la vis de serrage (5), des saillies distales également orientées vers l'extérieur dans le plan radial, des saillies distales du serre-tige (6) également orientées vers l'extérieur dans le plan radial étant notamment prévues, ces saillies s'imbriquant dans les entailles radiales du serre-tige (6), le serre-tige (6) et l'adaptateur de serre-tige (6a) étant notamment soudés entre eux.

8. Dispositif selon l'une quelconque des revendications précédentes, **caractérisé en ce que** le serre-tige (6) comporte sur son côté opposé à la vis de serrage (5) un évidement de serrage (6.5) en forme de cercle partiel et/ou que la vis de serrage (5) comporte un filetage de vis (5.2) asymétrique.

9. Dispositif selon la revendication 8, **caractérisé en ce qu'**un flanc (5.4), orienté dans la direction de vissage à bloc (F) dans le plan proximal, du filetage (5.2) de la vis de serrage (5) s'étend selon un angle inférieur ou égal à 5°, notamment inférieur ou égal à 3°, jusqu'à un plan radial par rapport à l'axe de symétrie (S) de la vis de serrage (5).

10. Dispositif selon la revendication 8, **caractérisé en ce qu'**un flanc (5.3), orienté distalement dans une direction de vissage à bloc (F), du filetage (5.2) de la vis de serrage (5) s'étend selon un angle différent de 90° par rapport à l'axe de symétrie (S) de la vis de serrage (5), de préférence de 30°, par rapport à un plan radial.

11. Dispositif selon l'une quelconque des revendications précédentes, **caractérisé en ce que** la vis de serrage (5) comporte sur son côté opposé au serre-tige (6) un renfoncement (5.1) multiple ou non cylindrique.

12. Dispositif selon l'une quelconque des revendications précédentes, **caractérisé en ce que** l'élément de compression (9, 9a) est disposé de façon à pouvoir basculer dans la tulipe (3).

13. Dispositif selon la revendication 12, **caractérisé en ce que** le diamètre de l'élément de compression (9, 9a) est réduit au niveau d'au moins un de ses côtés avant par rapport au diamètre intérieur de la tulipe (3), de sorte que l'élément de compression (9, 9a) puisse basculer à l'intérieur de la tulipe (3).

14. Dispositif selon l'une quelconque des revendications 12 ou 13, **caractérisé en ce que** l'élément de compression (9) de la tulipe (3) se rétrécit depuis son côté distal vers son côté avant, de façon à obtenir dans le plan proximal un jeu radial (9.6) entre la paroi intérieure de la tulipe (3) et l'enveloppe (9.4) de l'élément de compression (9) ou que la paroi intérieure de la tulipe (3) s'élargit depuis la hauteur du côté avant proximal jusqu'à la hauteur du côté avant distal de l'élément de compression (9a) en garantissant un jeu radial (9.6) entre l'élément de compression (9a) et la tulipe (3) jusqu'à atteindre la hauteur du côté avant distal de l'élément de compression (9a).

15. Dispositif selon l'une quelconque des revendications précédentes, **caractérisé en ce que** la tige de vis (2.1) de la vis pédiculaire (2) comporte un double filetage (2.2), une zone proximale de la tige de vis (2.1) étant notamment réalisée sous la forme d'un quadruple filetage (2.2.1), de préférence sur une longueur d'un quart de la tige de vis pédiculaire (2.1).
